(19) Europäisches Patentamt
European Patent Office
Office européen des brevets

(11) EP 0 983 259 B1

(12) **FASCICULE DE BREVET EUROPEEN**

(45) Date de publication et mention de la délivrance du brevet:
**07.08.2002 Bulletin 2002/32**

(21) Numéro de dépôt: **98925707.6**

(22) Date de dépôt: **14.05.1998**

(51) Int Cl.[7]: **C07D 311/72**, A61K 31/355, A61K 7/48

(86) Numéro de dépôt international:
**PCT/FR98/00958**

(87) Numéro de publication internationale:
**WO 98/51679 (19.11.1998 Gazette 1998/46)**

(54) **ESTERS DE TOCOPHEROL ET LEURS UTILISATIONS EN COSMETIQUE ET PHARMACIE**

TOCOFEROL ESTERN UND DEREN VERWENDUNG IN KOSMETIKA UND PHARMAZIE

TOCOPHEROL ESTERS AND THEIR COSMETIC AND PHARMACEUTICAL USES

(84) Etats contractants désignés:
**DE ES FR GB IT**

(30) Priorité: **14.05.1997 FR 9705907**

(43) Date de publication de la demande:
**08.03.2000 Bulletin 2000/10**

(73) Titulaire: **LVMH RECHERCHE**
**75008 Paris (FR)**

(72) Inventeurs:
- **BONTE, Frédéric**
  **F-45100 Orléans (FR)**
- **SAUNOIS, Alex**
  **45240 Menestreau en Villette (FR)**

(74) Mandataire: **Portal, Gérard**
**Cabinet Beau de Loménie**
**158, rue de l'Université**
**75340 Paris Cédex 07 (FR)**

(56) Documents cités:
**DE-A- 2 439 139** **FR-A- 2 314 722**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

DOMAINE DE L'INVENTION

L'invention concerne à titre de produit nouveau des esters de tocophérol, une utilisation cosmétique ou pharmaceutique, notamment dermatologique de ceux-ci, ainsi que des compositions cosmétiques ou pharmaceutiques, notamment dermatologiques les contenant.

PRESENTATION DE L'ART ANTERIEUR

On sait que l'alpha-tocophérol ou vitamine E se trouve à l'état naturel dans de nombreuses plantes, habituellement avec d'autres composés tels que le béta-tocophérol, le gamma-tocophérol ou le delta-tocophérol.

L'alpha-tocophérol est essentiellement utilisé pour lutter contre les déficiences en vitamine E, ou comme facteur nutritionnel, notamment pour lutter contre la dégénérescence musculaire.

Il est également utilisé comme anti-oxydant, mais à des doses très spécifiques.

PRESENTATION DE L'INVENTION

Dans le cadre de la présente invention, il a maintenant été découvert, de manière tout à fait surprenante et inattendue, des nouveaux esters de tocophérol, et le fait que ces nouveaux esters de tocophérol présentent une puissante activité anti-radicalaire, anti-inflammatoire, d'amélioration de la différenciation des kératinocytes, d'amélioration de l'hydratation cutanée, d'amélioration de la finesse des grains de la peau, une activité anti-vieillissement, une activité dépigmentante ainsi qu'une action anesthésique sur les terminaisons nerveuses cutanées.

Ainsi, la présente invention a pour but de résoudre le nouveau problème technique consistant en la fourniture d'une substance active présentant une bonne activité anti-radicalaire, anti-inflammatoire, anti-vieillissement, dépigmentante, améliorant la différenciation des kératinocytes, l'hydratation cutanée, la finesse du grain de la peau, ainsi qu'une action anesthésique sur les terminaisons nerveuses cutanées, en particulier par voie topique ou générale, en constituant ainsi un ingrédient actif précieux pour la préparation d'une composition cosmétique, ou pharmaceutique, notamment dermatologique.

La présente invention résout ce nouveau problème technique de manière satisfaisante, selon une solution particulièrement simple, utilisable à l'échelle industrielle.

DESCRIPTION DETAILLEE DE L'INVENTION

Ainsi, selon un premier aspect, la présente invention couvre des esters, caractérisés en ce qu'ils présentent la formule chimique suivante (I)

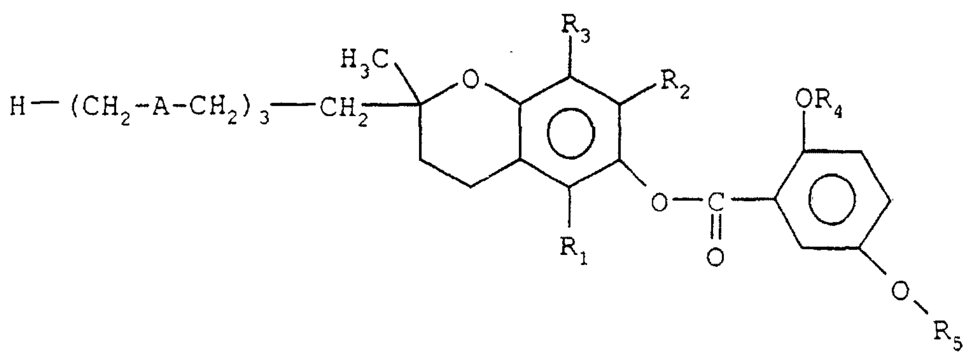

(I)

dans laquelle :

- $R_1$, $R_2$ et $R_3$ représentent indépendamment un atome d'hydrogène ou un radical méthyle,
- A représente les groupes :

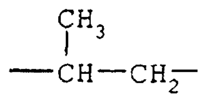 , ou 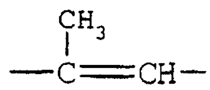 ,

$R_4$ et $R_5$ sont identiques ou différents et représentent chacun un enchaînement de la forme :

$$-B_m-C_n-B_p-C_q-H$$

dans lequel
B est un groupement

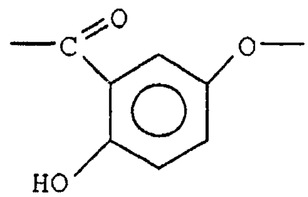

C est un groupement

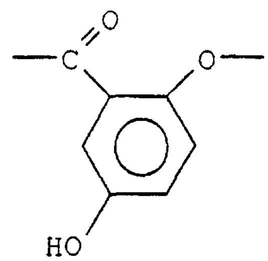

et chacun des indices m, n, p et q est un entier compris entre 0 et 4, étant entendu que la somme m + n + p + q est limitée aux nombres entiers de 0 à 4.

Suivant une variante de réalisation de l'invention, les esters sont caractérisés en ce que $R_4$ et $R_5$ représentent l'hydrogène.

Suivant une variante de réalisation de l'invention, les esters sont caractérisés en ce qu'au moins un des groupements $R_4$ et $R_5$ représente l'hydrogène.

Suivant une variante de réalisation de l'invention, les esters sont caractérisés en ce que l'un des groupements $R_4$ ou $R_5$ représente l'hydrogène et l'autre un radical 2,5-dihydroxybenzoyle.

Suivant une variante de réalisation de l'invention, les esters sont caractérisés en ce qu'ils sont choisis parmi le groupe consistant des esters de l'alpha-tocophérol, du béta-tocophérol, du gamma-tocophérol, du dzêta 1 tocophérol, du dzêta 2-tocophérol, du delta-tocophérol, de l'êta-tocophérol, de l'epsilon-tocophérol et du tocol.

Suivant une variante de réalisation de l'invention, celle-ci concerne un ester répondant à la formule suivante (II):

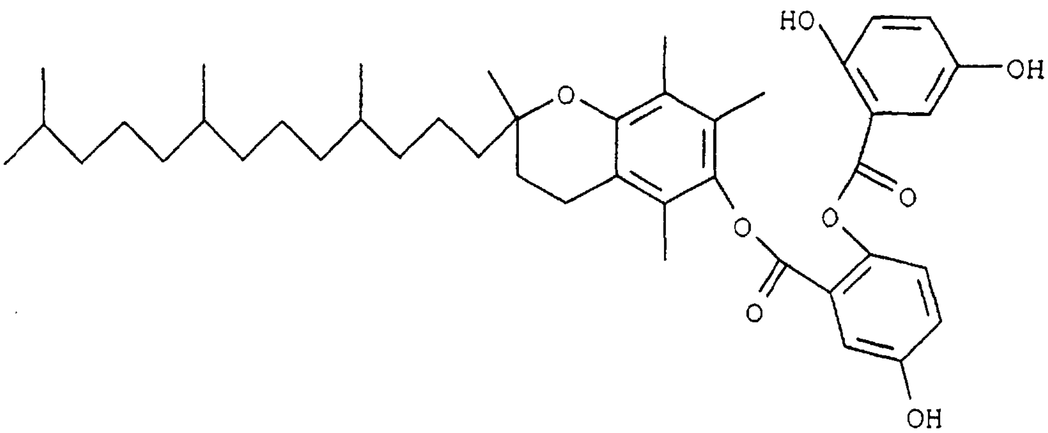

(II)

Suivant une variante de réalisation de l'invention, celle-ci concerne un ester répondant à la formule suivante (III) :

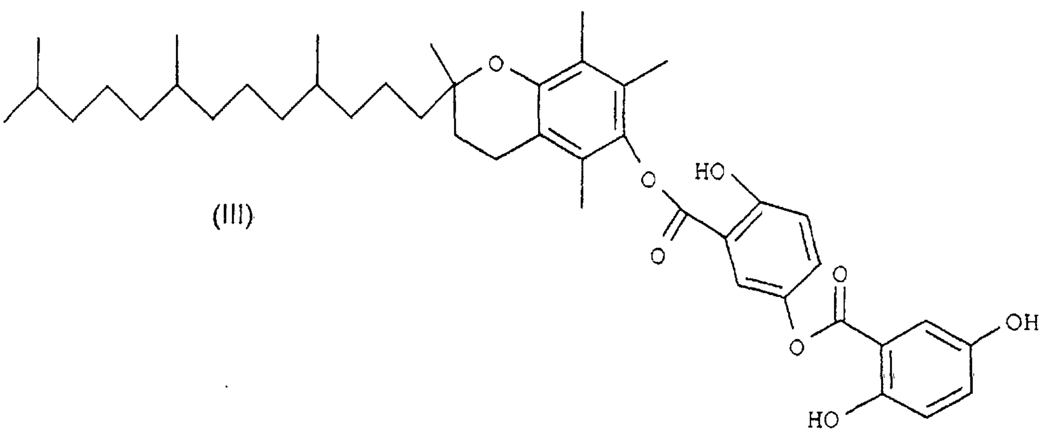

(III)

Les esters de formules (II) et (III) ci-dessus sont désignés ci-après par l'expression diester de 2,5-dihydroxybenzoate d'alpha-tocophérol, respectivement de formule (II) et de formule (III), ou encore "diester $A_1$" et "diester $A_2$".
Selon un deuxième aspect, la présente invention couvre aussi une composition cosmétique ou pharmaceutique, notamment dermatologique, caractérisée en ce qu'elle comprend comme ingrédient actif au moins un ester de formule (I), tel que précédemment défini.
Selon une variante de réalisation, la composition précitée est caractérisée en ce qu'elle comprend comme ingrédient actif au moins deux esters tels que précédemment définis, sous forme de mélange.
Selon une variante de réalisation, la composition précitée est caractérisée en ce que l'ester précité est présent dans une phase grasse de ladite composition.
Selon encore une variante de réalisation, la composition précitée est caractérisée en ce que la phase grasse comprend au moins une huile cosmétiquement ou pharmaceutiquement ou dermatologiquement acceptable, en particulier choisie parmi le groupe constitué d'huile de jojoba, d'huile de macadamia, d'huile de graine de limnanthe, en particulier Limnanthes alba Benth, en anglais Meadowfoam, d'huiles minérales et de triglycéride, en particulier de triglycéride à base d'acide caprilique et/ou caprique, ou de leurs mélanges.
Selon une variante de réalisation, la composition précitée est caractérisée en ce que la concentration en ester de formule I précité est comprise entre 0,001 et 5 %, encore mieux de 0,01 à 1 % en poids par rapport au poids total de la composition finale.
Selon une variante particulière de réalisation, la composition précitée est caractérisée en ce qu'elle comprend comme ingrédient actif au moins un ester d'alpha-tocophérol correspondant à la formule chimique I précitée, dans laquelle $R_1$, $R_2$ et $R_3$ représentent chacun un radical méthyle,

A représente le groupe

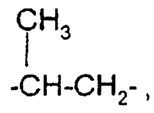

$R_4$ et $R_5$ ont les valeurs précédemment définies.

Selon une autre variante de réalisation, la composition précitée est caractérisée en ce qu'elle comprend au moins un autre ingrédient actif, en particulier choisi parmi le groupe constitués par les vitamines A, B1, B6, B12, E, C, PP, l'acide rétinoïque, le rétinal, le rétinol et ses esters, l'acide salicylique et ses dérivés, en particulier ses sels ou ses esters, l'acide 2,5-dihydroxy-benzoïque, le tocophérol et ses esters, en particulier le phosphate de tocophérol, l'acide asiatique, l'acide madécassique et ses esters glycosylés, l'asiaticoside, un extrait de Centella asiatica, un extrait de Siegesbeckia orientalis, les oligomères proantocyanidiques notamment ceux obtenus à partir de pépins de raisin, et leurs esters, en particulier palmitique et stéarique, les dérivés d'acide ascorbique, en particulier le phosphate et ses sels, l'acide erythorbique, les oligoéléments notamment sous forme de sel, en particulier sous forme d'aspartate ou de chlorure, tel que magnésium, sélénium, zinc ou cuivre, les acides alpha-hydroxylés, notamment l'acide malique, l'acide lactique et l'acide tartrique, et leurs esters, en particulier avec les alcools gras, tels que l'alcool stéarylique, les acides aminés, en particulier la sérine, la thréonine, la citrulline, ainsi que les acides aminés constituants du NMF, (Natural Moisturizing Factor : K. Sakamoto, Cosmet. Toilet. (1984) 99 (3) 109-117), les céramides, en particulier les céramides 2, 3 ou 6 utilisés seuls ou en mélange, les phytocéramides en particulier ceux extraits du blé et les ecdystéroïdes en particulier l'ecdystérone, et leurs esters.

Selon une variante particulière de réalisation, la composition précitée est caractérisée en ce que l'ester de formule I précité est utilisé en combinaison avec la vitamine A, de préférence sous forme d'un ester tel que palmitate.

Selon un troisième aspect, la présente invention couvre aussi l'utilisation comme agent cosmétique d'au moins un ester de formule I tel que précédemment défini, avantageusement inclus dans la composition cosmétique précitée.

Dans le cadre de son utilisation comme agent cosmétique, l'ester de formule (I) précité est utilisé pour éviter ou atténuer les effets néfastes des radicaux libres sur la peau, pour prévenir ou traiter les rougeurs et les sensations de picotement ou de brûlures de la peau, pour favoriser la différenciation des kératinocytes, pour restaurer l'hydratation normale de l'épiderme, pour améliorer la finesse du grain de la peau, pour ralentir ou traiter les effets du vieillissement sur la peau, tels que l'apparition des rides ou le relâchement cutané, et pour atténuer l'hyperpigmentation, notamment les taches pigmentaires dues à la sénescence cutanée.

Parmi les effets néfastes des radicaux libres, il en est un tout particulièrement dommageable dû aux radicaux libres constitués d'oxygène, qui est la peroxydation des substances cutanées, en particulier des lipides membranaires des cellules, telles que les kératinocytes. Ces radicaux libres constitués d'oxygène sont en proportion de plus en plus grandes dans des atmosphères polluées, en raison de l'action combinée de la température, du soleil, et des polluants industriels, incluant les gaz d'échappement des véhicules automobiles. Ces radicaux libres provoquent un vieillissement accéléré de la peau, dont les effets peuvent être prévenus ou traités ou atténués par l'utilisation des esters de la présente invention.

Selon un quatrième aspect, la présente invention couvre aussi l'utilisation d'au moins un ester de formule I tel que précédemment défini pour la fabrication d'une composition pharmaceutique, notamment dermatologique, à activité anti-radicalaire, anti-inflammatoire, ou à action anesthésique sur les terminaisons nerveuses cutanées.

Selon une variante, ladite composition comprend de 0,001 à 5 %, encore mieux de 0,01 à 1 % en poids de la composition finale.

Selon un cinquième aspect, la présente invention couvre encore un procédé de traitement cosmétique, caractérisé en ce qu'on applique topiquement sur la peau d'un être humain, une quantité cosmétiquement efficace d'au moins un ester de formule I tel que précédemment défini, en particulier sous forme d'une composition contenant l'ester de formule I précité à une concentration comprise entre 0,001 et 5 %, et de préférence entre 0,01 et 1 %, en poids de la composition finale.

Il ressort de la description précédente que les compositions selon l'invention peuvent être formulées selon toute forme acceptable pour une utilisation en cosmétologie, dermatologie ou pharmacie. Il peut, en particulier, s'agir d'une crème, d'une lotion ou d'une émulsion ou encore d'une lotion.

L'invention sera maintenant illustrée en détail à l'aide de plusieurs exemples de réalisation donnés simplement à titre d'illustration et qui ne sauraient donc en aucune façon limiter la portée de l'invention, en référence aussi à la figure unique annexée.

PRESENTATION DE LA FIGURE UNIQUE ANNEXEE

La figure unique annexée rapporte en abscisse les concentrations en peroxyde d'hydrogène (H2O2) utilisé comme générateur de radicaux libres créant la toxicité cellulaire, qui est éventuellement combattue par le DHBT selon l'invention, à la concentration testée, tandis que l'ordonnée rapporte en pourcentage la viabilité cellulaire, telle qu'obtenue par le test rouge neutre sur les kératinocytes humains normaux ou KHn.

Dans les exemples, les pourcentages sont donnés en poids, la température est la température ambiante, et la pression est la pression atmosphérique, sauf indication contraire. En cas d'indication de température, celle-ci est indiquée en degré Celsius.

Exemple 1 : Synthèse d'esters de l'acide 2,5-dihydroxybenzoïque avec l'alpha-tocophérol

Le principe réactionnel consiste à faire réagir l'acide 2,5-dihydroxybenzoïque sur l'alpha-tocophérol, ce qui conduit à la formation d'esters de formule chimique I précitée, dans laquelle :

A représente le groupe

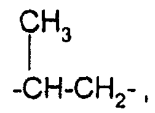

$R_1$, $R_2$ et $R_3$. représentent chacun un radical méthyle
$R_4$ et $R_5$ ont les valeurs précédemment définies.

Plus précisément, la synthèse des esters est réalisée de la manière suivante :

On charge dans un ballon de 2 litres équipé d'une agitation demi-lune, d'un thermomètre, d'un réfrigérant et d'un Dean Starck, 172,3 g (0,4 mole) d'alpha-tocophérol, 600 g de xylène, 123,2 g (0,8 mole) d'acide 2,5-dihydroxybenzoique.
On additionne à 25°C 56 g d'acide sulfurique à 96 %, ce qui produit une réaction exothermique.
On réalise une distillation de l'eau formée pendant une durée d'environ 7 h. On refroidit à 50°C, on émulsionne environ 1 000 g et ensuite on extrait au chloroforme en ajoutant 2 500 g de chloroforme.
On lave la phase organique avec 500 g de solution aqueuse de carbonate de sodium à 2 % et avec 1 500 g d'eau. On décante et on sépare la phase aqueuse.
On distille la phase organique à base de chloroforme et on obtient 203,4 g d'esters d'alpha-tocophérol avec un rendement de 89 %. Les esters d'alpha-tocophérol se présentent sous forme d'un liquide visqueux brun foncé. Selon un dosage par analyse en chromatographie haute performance CLHP, d'après les rapports de surface, ce liquide comprend 6,3 % de tocophérol, 49 % d'esters de 2,5-dihydroxybenzoate d'alpha-tocophéryle et 34 % d'autres produits.

Procédé de purification

Dans un ballon de 2 litres équipé d'une agitation demi-lune, d'un thermomètre, d'un réfrigérant, on charge 180 g du liquide visqueux contenant les esters de 2,5-dihydroxybenzoate d'alpha-tocophéryle, obtenu à l'étape précédente, ainsi que 600 g de toluène, puis du charbon actif sous forme pulvérulente.

Après une période de contact avec du charbon actif de plusieurs heures, on clarifie en filtrant le charbon actif puis on distille le toluène. On obtient ainsi 116 g d'esters de 2,5-dihydroxybenzoate d'alpha-tocophéryle,. Le rendement est de 64 %. Ces esters de 2,5-dihydroxybenzoate d'alpha-tocophéryle se présentent sous forme d'un liquide visqueux brun.

Un nouveau dosage par analyse CLHP donne 10,4 % d'alpha-tocophérol, 54 % d'esters de 2,5-dihydroxybenzoate d'alpha-tocophéryle et 31 % d'autres produits. L'analyse confirme que ces esters sont des 2,5-dihydroxybenzoates d'alpha-tocophérol.

On observera que cet exemple qui est donné avec l'alpha-tocophérol peut être réalisé sans aucune difficulté avec d'autres tocophérols.

Exemple 2 : Préparation du mono-ester de 2,5-dihydroxybenzoate d'alpha-tocophérol purifié

On peut obtenir le mono-ester de 2,5-dihydroxybenzoate d'alpha-tocophérol avec un bon rendement, en utilisant les conditions de synthèse décrites à l'exemple 1, mais en réalisant une réaction ménagée à température plus basse, à savoir de l'ordre de 40 à 60° et avec un temps de réaction plus court, à savoir de 2 à 4 h.

On obtient, dans ce cas, comme principal produit de réaction un mélange de mono-ester et de diesters de 2,5-dihydroxybenzoate d'alpha-tocophérol sous forme d'une solution chloroformique, notée ci-après SC. On peut séparer ces esters selon le procédé suivant :

A - Purification du mono-ester de 2,5-dihydroxybenzoate d'alpha-tocophérol

A partir de la solution chloroformique SC à 100 mg/ml, obtenue précédemment, on effectue une chromatographie silice préparative, par exemple sur plaque Merck F254, avec comme système éluant un mélange hexane/acétate d'éthyle (80/20 en volume). On récupère la fraction suivie par absorption UV à 320 nm. On concentre et on réduit à sec. On obtient un produit de rf 0,6 constitué par le mono-ester de 2,5-dihydroxybenzoate d'alpha-tocophérol.

Le mono-ester de 2,5-dihydroxybenzoate d'alpha-tocophérol se présente sous la forme d'un liquide visqueux beige clair, fluorescent sous UV courts et longs, soluble dans le chloroforme et éthanol.

Sa formule chimique brute est C36H54 O5 avec un poids moléculaire de 566 g.

Le spectre UV se caractérise par les λ max suivants : 330, 286, 277 nm.

Par spectrométrie de masse en impact électronique (70 eV), on obtient les fragments caractéristiques $M^+$ 566, 430, 416, 301.

Sa formule chimique développée est la suivante :

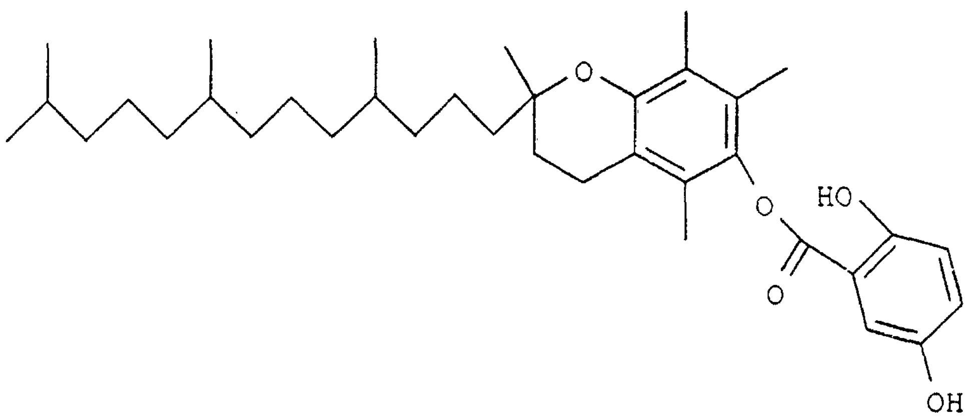

B - Procédé de purification de diesters de 2,5-dihydroxybenzoate d'alpha-tocophérol

A partir de la solution chloroformique SC précitée à 100 mg/ml, on effectue une chromatographie silice préparative, par exemple sur plaque Merck F254, avec comme système éluant un mélange hexane/acétate d'éthyle (80/20 en volume). On récupère une fraction majoritaire suivie par absorption UV à 320 nm que l'on concentre et que l'on réduit à sec.

On obtient un produit de rf 0,45 constitué par le mélange de deux diesters 2,5-dihydroxybenzoate d'alpha-tocophéryle, à savoir: le diester dans lequel le deuxième ester 2,5-dihydroxybenzoïque constitue un substituant ortho du premier ester, désigné "diester $A_1$" de formule (II) décrite plus haut, et le diester dans lequel le deuxième ester 2,5-dihydroxybenzoïque est un substituant en position méta du premier ester, désigné "diester $A_2$" de formule (III) décrite plus haut.

Les caractéristiques du mélange des diesters de 2,5-dihydroxybenzoique d'alpha-tocophérol, respectivement de formules (II) et (III), sont les suivantes : rf 0,45 liquide visqueux ocre jaune léger, fluorescent sous UV courts et longs, soluble dans le chloroforme et éthanol. La formule chimique brute est C43H58 08, avec un poids moléculaire de 702, le spectre UV présente des λ max à 338, 286, 277 nm. La spectrométrie de masse en impact électronique (70 eV) donne les fragments caractéristiques $M^+$702, 670, 662, 647, 566, 430, 416,301.

A titre d'exemple, on peut séparer les diesters $A_1$ et $A_2$, en faisant subir au mélange brut une chromatographie sur plaque de silice C-18 (F254 de chez MERCK®), dans les conditions de solvant d'élution méthanol/eau/acide acétique/tétrahydrofurane dans un rapport en volume : 88,4/7,6/3,92/ 0,08.

Dans l'optique d'une séparation de ces deux diesters par chromatographie préparative industrielle sur colonne

de silice, on utilisera préférentiellement comme éluant un mélange hexane/acétate d'éthyle 95/5.

Le diester $A_1$ présente les intensités relatives des ions de fragmentation proches de l'ion moléculaire (702) suivantes : 702 (10), 670 (70), 662 (14), 647 (22).

Le diester $A_2$ présente les intensités relatives des ions de fragmentation proches de l'ion moléculaire (702) suivantes : 702 (3), 670 (25), 662 (28), 647 (48).

Exemple 3 : Etude de l'effet protecteur des esters de 2,5-dihydroxybenzoate d'alpha-tocophérol, vis-à-vis de la cytotoxicité des radicaux libres

1°. Principe du test

Différentes concentrations de 2,5-dihydroxybenzoate d'alpha-tocophérol, ci-après désigné "DHBT", dissous dans du DMSO (diméthylsulfoxyde), sont testées dans des cultures de kératinocytes humains normaux (KHn) exposés à différentes concentrations de radicaux libres oxygénés produits par du peroxyde d'hydrogène ($H_2O_2$) présent dans les milieux de culture.

Pour chaque concentration testée, la viabilité cellulaire est évaluée par le test dit "au rouge neutre".

Le test comprend également des essais comparatifs, à savoir :

- des cultures témoins ne recevant pas de peroxyde d'hydrogène mais recevant comme les cultures traitées du DHBT dissous dans le DMSO (témoin blanc),
- des cultures ne recevant pas de peroxyde d'hydrogène, mais recevant du DMSO, solvant du DHBT, en quantité identique à celle des cultures traitées par le DHBT (témoin DMSO).

Le DHBT testé, dissous dans le DMSO, est en fait le mélange d'esters de 2,5-dihydroxybenzoate d'alpha-tocophérol obtenu à l'exemple 1.

2°. Protocole du test

a) Provenance des kératinocytes

On utilise des kératonocytes humains normaux (KHn), qui ont été prélevés par voie chirurgicale à partir de peaux saines.

b) Conditions de culture

Les KHn sont maintenus dans les milieux SFM (Serum Free Medium) complet (noté SFMc, Gibco). Les cellules ont été sous cultivées successivement sept fois et au septième passage, noté P7, les cellules de KHn sont récoltées.

c) Conditions de traitement

L'ensemencement des cellules de KHn P7 est réalisé en plaques de cultures à 96 puits à raison de 10 00 KHn par puits en milieu sans sérum, milieu K-SFM de chez Gibco, et traités 24 heures plus tard avec des concentrations différentes de peroxyde d'hydrogène (H2O2, Sigma, référence H-1009), respectivement de 1 µM, 5 µM, 10 µM, 25 µM, 50 µM, 75 µM, 100 µM. Pour chacune de ces concentrations de H2O2, trois concentrations de gentisate de tocophérol sont également testées, respectivement à 0,1 µg/ml, 1 µg/ml, 10 µg/ml.

L'incubation des cellules KHn avec les différents produits est réalisée dans une solution saline équilibrée de HANKS (HBSS, de Gibco, référence : 14175-053), pendant une heure à 37°C. Après incubation, les cellules sont rincées avec du tampon HBSS, et un test au rouge neutre est réalisé, qui constitue un test de viabilité, mettant en évidence une activité lysosomiale.

d) Test au rouge neutre

Ce test consiste à réaliser un rinçage des cellules au tampon phosphate de Dulbecco (PBS, chez Gibco, Référence: 14190).

Ensuite, on réalise une incubation des cellules pendant 3 heures à 37°C avec 200 µl par puits d'une solution de rouge neutre (solution mère à 0,4 % dans l'eau) à 1,25 % (v/v) dans un milieu SFMc.

Seules les cellules vivantes se colorent en rouge. Par conséquent, l'intensité de la couleur rouge, mesurée

en densité optique, dépend directement du nombre de cellules vivantes.

Après incubation, on réalise un rinçage des cellules au PBS.

Ensuite, on ajoute 100 $\mu$l par puits d'un mélange éthanol 50 % + acide acétique 1 %.

On agite la plaque pendant 15 minutes.

Enfin, on réalise par spectrophotométrie une lecture de densité optique à 540 nm.

La viabilité cellulaire est exprimée en pourcentage, suivant la formule ci-après :

$$\text{viabilité (\%)} = \frac{DO_{PH}}{DO_{TB}} \times 100$$

dans laquelle, pour chaque valeur de concentration de DHBT, $DO_{TB}$ est la densité optique des cultures "témoin blanc", et $DO_{PH}$ est la densité optique des cultures contenant le peroxyde d'hydrogène $H_2O_2$.

3°. Résultats

Les résultats obtenus sont répertoriés au tableau I ci-après :

## TABLEAU I

Viabilité (%) des kératinocytes $KH_n$ en culture, en présence de 2,5-dihydroxybenzoate d'alpha-tocophérol (DHBT) et de radicaux libres oxygénés

| | Absence de DHBT | | DHBT 0,1 µg/ml | | DHBT 1 µg/ml | | DHBT 10 µg/ml | |
|---|---|---|---|---|---|---|---|---|
| | Moyenne | σ | Moyenne | σ | Moyenne | σ | Moyenne | σ |
| Témoin blanc | 100,00 | 6,82 | 100 | 2,82 | 100 | 2,45 | 100 | 4,21 |
| Témoin DMSO | | | 107,38** | 1,11 | 103,85** | 1,04 | 105,31** | 3 |
| H2O2 1µM | 108,25 | 8,13 | 110,47 | 3,66 | 99,88 | 1,36 | 103,67 | 3,66 |
| H2O2 5 µM | 106,93 | 6,2 | 109,88 | 0,45 | 106,87 | 0,67 | 107,67 | 3,11 |
| H2O2 10 µM | 109,04 | 8,44 | 102,85 | 8,47 | 103,74 | 2,26 | 111,99 | 2,44 |
| H2O2 25 µM | 95,16 | 6,39 | 94,16 | 20,07 | 99,21 | 4,49 | 112,35 | 4,59 |
| H2O2 50 µM | 85,33* | 4,74 | 85,1* | 2,75 | 86,52* | 4,32 | 107,04 | 4,93 |
| H2O2 75 µM | 72,42* | 11,4 | 85,37* | 2,65 | 72,46* | 3,32 | 90,73* | 4,41 |
| H202 100 µM | 59,07* | 8,9 | 68,4* | 4,08 | 53,04* | 6,87 | 85,71* | 2,43 |

* résultats significatifs à 5 % ($p < 0,05$)

** les cultures témoins DMSO ne reçoivent pas le DHBT mais uniquement le DMSO

Ces résultats sont aussi rapportés sous forme de courbes à la figure unique annexée.

En abscisse, on rapporte les concentrations en peroxyde d'hydrogène (H2O2) utilisé comme générateur de radicaux libres créant la toxicité cellulaire, qui est éventuellement combattue par le DHBT selon l'invention, à la concentration testée, tandis que l'ordonnée rapporte en pourcentage la viabilité cellulaire, telle qu'obtenue par le test rouge neutre sur les kératinocytes humains normaux ou KHn.

Parmi ces courbes, la courbe reliant les ronds correspond aux cultures contenant du peroxyde d'hydrogène, mais ne recevant pas de DHBT ; la courbe qui relie des carrés correspond aux cultures recevant le DHBT dans le DMSO selon l'invention à une concentration de 0,1 µg/ml de culture ; la courbe qui relie des points en forme de triangle correspond aux cultures recevant le DHBT dans le DMSO, selon l'invention à une concentration de 1 µg/ml de culture, et enfin la courbe qui réunit des points en forme de croix correspond aux cultures recevant le DHBT selon l'invention à une concentration de 10 µg/ml de culture.

On rappellera que les cultures correspondant au témoin DMSO ne reçoivent pas le DHBT mais uniquement son solvant DMSO.

Il ressort du tableau I, ainsi que des courbes rapportées à la figure unique annexée, que le peroxyde d'hydrogène est toxique sur les kératinocytes utilisés à partir de 25 µM, à partir de laquelle le taux de viabilité cellulaire chute drastiquement depuis environ 100 %.

Cependant, lorsque les cellules sont traitées à la fois par le peroxyde d'hydrogène et le DHBT selon l'invention à une concentration de 10 µg/ml, la toxicité des peroxydes d'hydrogène ne peut être observée qu'à partir de la concentration de 75 µM, c'est-à-dire pour une concentration trois fois supérieure, ce qui démontre une résistance significative des kératinocytes face à une attaque des radicaux libres grâce au DHBT, à une telle concentration.

Ainsi, cet essai permet bien de démontrer que l'activité anti-radicalaire est particulièrement utile notamment dans le cas de la fabrication d'une composition pharmaceutique, notamment dermatologique, ou cosmétique ou comme agent cosmétique.

En outre, étant donné que les radicaux libres oxygénés provoquent la peroxydation des lipides cutanés, la présente invention permet de prévenir ou de traiter efficacement les effets de vieillissement sur la peau. En particulier, ceux dus aux radicaux libres, et notamment ceux qui générés par la pollution atmosphérique.

L'invention sera maintenant décrite en référence à plusieurs exemples de compositions cosmétiques ou pharmaceutiques, notamment dermatologiques donnés simplement à titre d'illustration et qui ne sauraient donc aussi en aucune façon limiter la portée de l'invention.

Dans ces exemples, les concentrations sont données en gramme.

<u>Exemple 4 : crème antirides</u>

Cette crème est préparée à partir des ingrédients actifs suivants :

- esters de tocophérol purifié, selon l'exemple 1 0,1 g
- huile de jojoba 2 g
- huile de sésame 3 g
- vitamine A sous forme de palmitate 0,01 g
- glycérol 2 g
- eau + conservateur + tensioactif + parfum qsp 100 g

Cette crème est préparée de la façon suivante. On dissout tout d'abord les esters de tocophérol dans l'huile de jojoba et l'huile de sésame mélangées, puis on ajoute le palmitate de vitamine A, le glycérol et enfin la phase aqueuse avec le système tensioactif et l'on agite jusqu'à homogénéisation.

On obtient une crème qui, appliquée le soir pendant plusieurs semaines, affine le grain de la peau, donne un teint lumineux et prévient l'apparition des rides.

<u>Exemple 5 : composition anti-âge sous forme d'émulsion</u>

On prépare cette composition à partir des ingrédients actifs suivants :

- mélange des diesters 2,5-dihydroxybenzoate de tocophérol préparé à l'exemple 2 0,025 g
- stéarates de polyéthylèneglycol 6 et de polyéthylèneglycol 32 disponibles dans le commerce sous la référence commerciale TEFOSE 63® 15 g
- palmitate de cétyle 3 g
- alcool cétylique 3 g
- 2-hexyl-1-dodecanol 5 g

- glycérine 3 g
- gel de Carbopol 980® à 2 % 5 g
- eau purifiée + conservateur + parfum qsp 100 g

Pour la préparation de cette émulsion, on mélange tout d'abord le mélange des diesters 2,5-dihydroxybenzoate de tocophérol à la phase grasse jusqu'à dissolution complète, cette phase grasse étant constituée de la glycérine, des stéarates de polyéthylèneglycol, du palmitate de cétyle, de l'alcool de cétyle et de l'octyldodécanol. On ajoute ensuite l'eau purifiée sous agitation pour former une émulsion, puis on ajoute le gel de carbopol pour gélifier cette émulsion.

On obtient une composition formant émulsion ou crème que l'on applique localement pendant plusieurs semaines comme crème de soin anti-vieillissement.

Exemple 6 : composition sous forme de lotion dermatologique

Cette composition dermatologique est obtenue à partir des ingrédients actifs suivants :

- mélange des diesters 2,5-dihydroxybenzoate de tocophérol tel qu'obtenu à l'exemple 2 0,5 g
- éthanol absolu 35 g
- eau purifiée parfumée qsp 100 g

On prépare cette composition en mélangeant tout d'abord les diesters dans l'éthanol absolu, puis on ajoute l'eau purifiée sous agitation de manière à constituer ladite lotion.

Cette lotion appliquée sur la peau présente une action anti-vieillissement, affine le grain de la peau en lui donnant un teint lumineux.

**Revendications**

1. Esters, **caractérisés en ce qu'**ils présentent la formule chimique suivante (I):

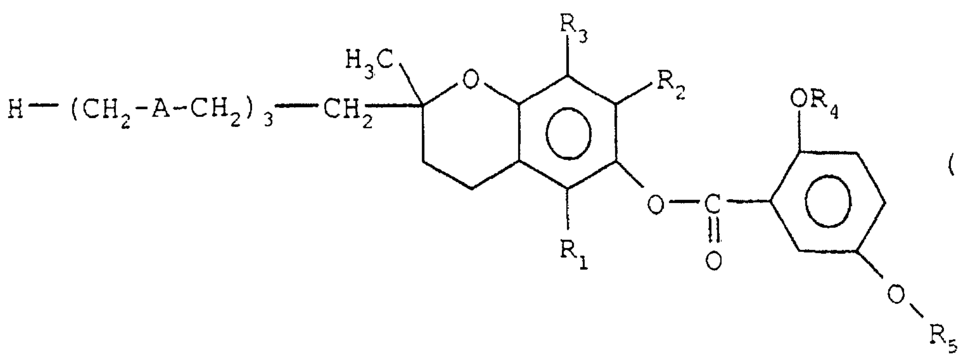

(I)

dans laquelle :

$R_1$, $R_2$ et $R_3$ représentent indépendamment un atome d'hydrogène, un radical méthyle,
A représente les groupes :

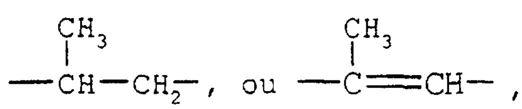

, ou ,

$R_4$ et $R_5$ sont identiques ou différents et représentent chacun un enchaînement de la forme :

$$-B_m-C_n-B_p-C_q-H$$

dans lequel
B est un groupement

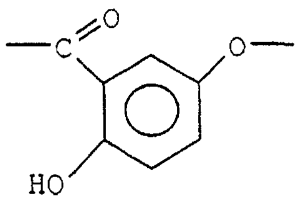

C est un groupement

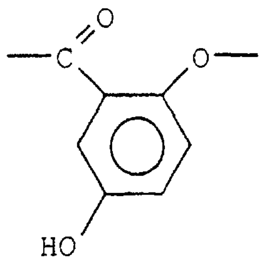

et chacun des indices m, n, p et q est un entier compris entre 0 et 4, étant entendu que la somme m + n + p + q est limitée aux nombres entiers de 0 à 4.

2. Esters selon la revendication 1, **caractérisés en ce que** $R_4$ et $R_5$ représentent l'hydrogène.

3. Esters selon la revendication 1, **caractérisés en ce qu'**au moins un des groupements $R_4$ et $R_5$ représente l'hydrogène.

4. Esters selon la revendication 3, **caractérisés en ce que** l'un des groupements $R_4$ ou $R_5$ représente l'hydrogène et l'autre un radical 2,5-dihydroxybenzoyle.

5. Esters selon l'une des revendications précédentes, **caractérisés en ce qu'**il est choisi parmi le groupe consistant des esters de l'alpha-tocophérol, du béta-tocophérol, du gamma-tocophérol, du dzêta 1-tocophérol, du dzêta 2 tocophérol, du delta-tocophérol, de l'êta-tocophérol, de l'epsilon-tocophérol et du tocol.

6. Ester, répondant à la formule (II) suivante :

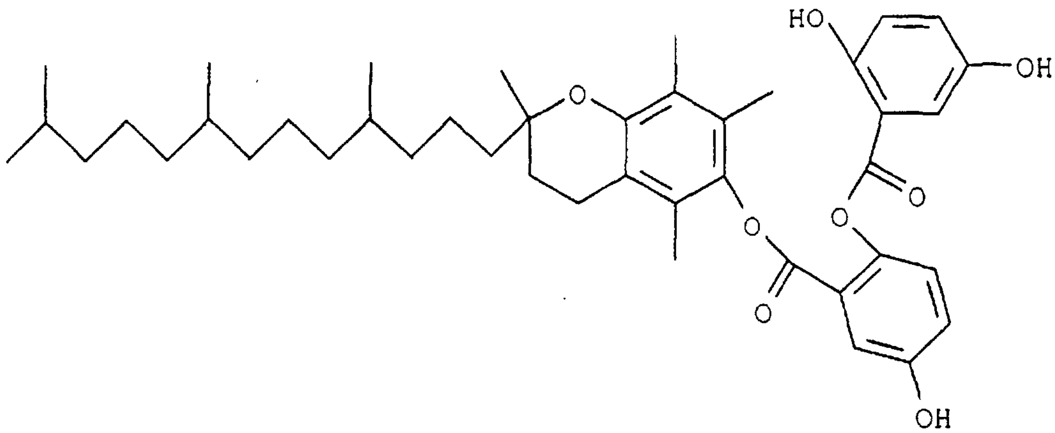

(II)

**7.** Ester, répondant à la formule (III) suivante :

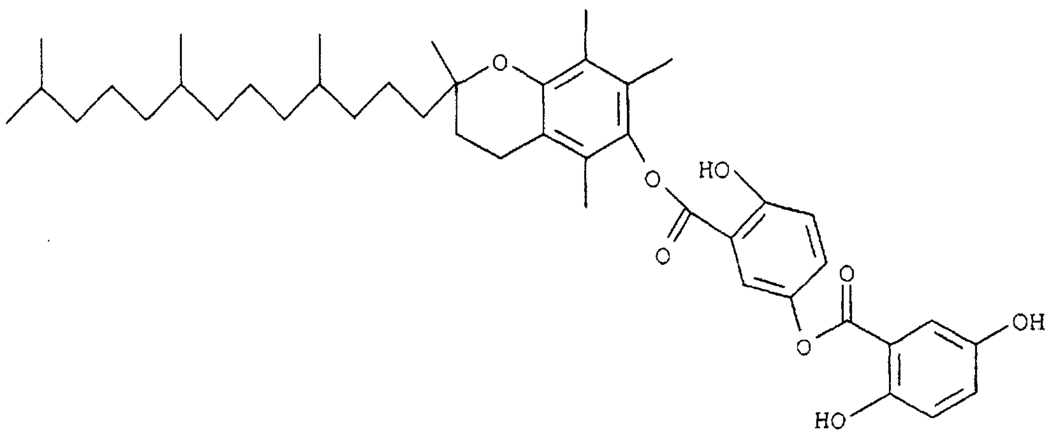

(III)

**8.** Composition cosmétique ou pharmaceutique, notamment dermatologique, **caractérisée en ce qu'**elle comprend comme ingrédient actif au moins un ester tel que défini à l'une quelconque des revendications précédentes.

**9.** Composition selon la revendication 8, **caractérisée en ce qu'**elle comprend comme ingrédient actif au moins deux esters tels que définis à l'une quelconque des revendications 1 à 7, sous forme de mélange.

**10.** Composition selon la revendication 9, **caractérisée en ce que** la phase grasse comprend au moins une huile cosmétiquement ou pharmaceutiquement ou dermatologiquement acceptable, en particulier choisie parmi le groupe constitué d'huile de jojoba, d'huile de macadamia, d'huile de graine de limnanthe, en particulier Limnanthes alba Benth, d'huiles minérales et de triglycérides, en particulier de triglycérides à base d'acide caprilique et/ou caprique, ou de leurs mélanges.

**11.** Composition selon l'une des revendications 8 à 10, **caractérisée en ce que** la concentration en ester est comprise entre 0,001 et 5 %, encore mieux de 0,01 à 1 % en poids par rapport au poids total de la composition finale.

**12.** Composition selon l'une des revendications 8 à 11, **caractérisée en ce qu'**elle comprend comme ingrédient actif au moins un ester d'alpha-tocophérol, correspondant à la formule chimique I, définie à la revendication 1, dans laquelle $R_1$, $R_2$ et $R_3$ représentent chacun un radical méthyle,

A représente le groupe

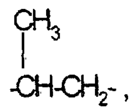

$R_4$ et $R_5$ tels que définis à l'une des revendications 1 à 4.

**13.** Composition selon l'une des revendications 8 à 12, **caractérisée en ce qu'**elle comprend au moins un autre ingrédient actif, en particulier choisi parmi le groupe constitué par les vitamines A, B1, B6, B12, E, C, PP, l'acide rétinoïque, le rétinal, le rétinol et ses esters, l'acide salicylique et ses dérivés, en particulier ses sels ou ses esters, l'acide 2,5-dihydroxy-benzoïque, le tocophérol et ses esters, en particulier le phosphate de tocophérol, l'acide asiatique, l'acide madécassique et ses esters glycosylés, l'asiaticoside, un extrait de Centella asiatica, un extrait de Siegesbeckia orientalis, les oligomères proantocyanidiques notamment ceux obtenus à partir de pépins de raisin, et leurs esters, en particulier palmitique et stéarique, les dérivés d'acide ascorbique, en particulier le phosphate et ses sels, l'acide erythorbique, les oligoéléments notamment sous forme de sel, en particulier sous forme d'aspartate ou de chlorure, tel que magnésium, sélénium, zinc ou cuivre, les acides alpha-hydroxylés, notamment l'acide malique, l'acide lactique et l'acide tartrique, et leurs esters, en particulier avec les alcools gras, tels que l'alcool stéarylique, les acides aminés, en particulier la sérine, la thréonine, la citrulline, ainsi que les acides aminés constituants du NMF, les céramides, en particulier les céramides 2, 3 ou 6 utilisés seuls ou en mélange, les phytocéramides en particulier ceux extraits du blé et les ecdystéroïdes en particulier l'ecdystérone, et leurs esters.

**14.** Composition selon l'une des revendications 8 à 13, **caractérisée en ce que** ledit ester est utilisé en combinaison avec la vitamine A, de préférence sous forme d'un ester tel que palmitate.

**15.** Utilisation d'au moins un ester tel que défini à l'une quelconque des revendications 1 à 7 comme agent cosmétique.

**16.** Utilisation d'au moins un ester tel que défini à l'une quelconque des revendications 1 à 7 comme agent cosmétique pour éviter ou atténuer les effets néfastes des radicaux libres sur la peau, pour prévenir ou traiter les rougeurs et les sensations de picotement ou de brûlures de la peau, pour favoriser la différenciation des kératinocytes, pour restaurer l'hydratation normale de l'épiderme, pour améliorer la finesse du grain de la peau, pour ralentir ou traiter les effets du vieillissement sur la peau, tels que l'apparition des rides ou le relâchement cutané, et pour atténuer l'hyperpigmentation, notamment les taches pigmentaires dues à la sénescence cutanée.

**17.** Utilisation d'au moins un ester selon l'une quelconque des revendications 1 à 7 pour la fabrication d'une composition pharmaceutique, notamment dermatologique, à activité anti-radicalaire, anti-inflammatoire, ou à action anesthésique sur les terminaisons nerveuses cutanées.

**18.** Utilisation selon l'une des revendications 15 à 17, **caractérisée en ce que** ledit ester est utilisé sous forme d'une composition comprenant de 0,001 à 5 %, encore mieux de 0,01 à 1 % en poids de la composition finale.

**19.** Procédé de traitement cosmétique, **caractérisé en ce qu'**on applique topiquement sur la peau d'un être humain, une quantité cosmétiquement efficace d'au moins un ester tel que défini à l'une quelconque des revendications 1 à 7, en particulier sous forme d'une composition contenant l'ester précité à une concentration comprise entre 0,001 et 5 %, et de préférence entre 0,01 et 1 %, en poids de la composition finale, en particulier pour éviter ou atténuer les effets néfastes des radicaux libres sur la peau, pour prévenir ou traiter les rougeurs et les sensations de picotement ou de brûlures de la peau, pour favoriser la différenciation des kératinocytes, pour restaurer l'hydratation normale de l'épiderme, pour améliorer la finesse du grain de la peau, pour ralentir ou traiter les effets du vieillissement sur la peau, tels que l'apparition des rides ou le relâchement cutané, et pour atténuer l'hyperpigmentation, notamment les taches pigmentaires dues à la sénescence cutanée.

## Patentansprüche

**1.** Ester, **dadurch gekennzeichnet, dass** sie die folgende chemische Formel (I) aufweisen:

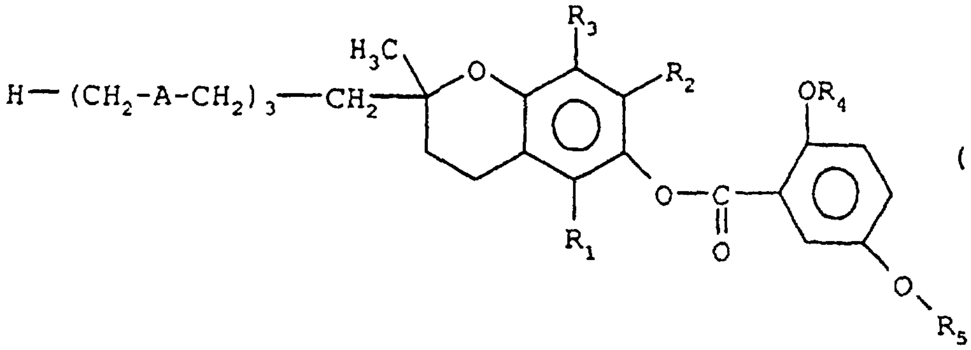

(I) (I)

worin:

$R_1$, $R_2$ und $R_3$, unabhängig voneinander, ein Wasserstoffatom oder einen Methyl-Rest,
A die Gruppen:

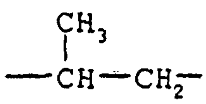

oder

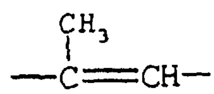

und
$R_4$ und $R_5$, gleich oder verschieden, jeweils eine Verkettung der Form darstellen:

$$-B_m-C_n-B_p-C_q-H$$

worin:

B eine Gruppierung:

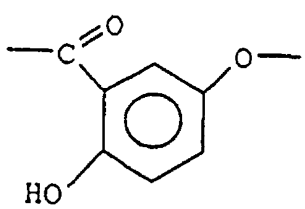

C eine Gruppierung:

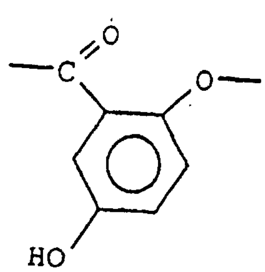

und jeder der Indizes m, n, p und q eine ganze Zahl von 0 bis 4 sind, wobei die Summe m + n + p + q jeweils eine ganze Zahl von 0 bis 4 beträgt.

2. Ester gemäß Anspruch 1, **dadurch gekennzeichnet, dass** $R_4$ und $R_5$ Wasserstoff darstellen.

3. Ester gemäß Anspruch 1, **dadurch gekennzeichnet, dass** mindestens eine der Gruppierungen $R_4$ und $R_5$ Wasserstoff darstellt.

4. Ester gemäß Anspruch 3, **dadurch gekennzeichnet, dass** eine der Gruppierungen Wasserstoff und die andere einen 2,5-Dihydroxybenzoyl-Rest darstellen.

5. Ester gemäß einem der vorhergehenden Ansprüche, **dadurch gekennzeichnet, dass** sie aus der Gruppe ausgewählt sind, bestehend aus Estern von α-Tocopherol, β-Tocopherol, γ-Tocopherol, Z-1-Tocopherol, Z-2-Tocopherol, δ-Tocopherol, E-Tocopherol, ε-Tocopherol und von Tocol.

6. Ester der folgenden Formel (II):

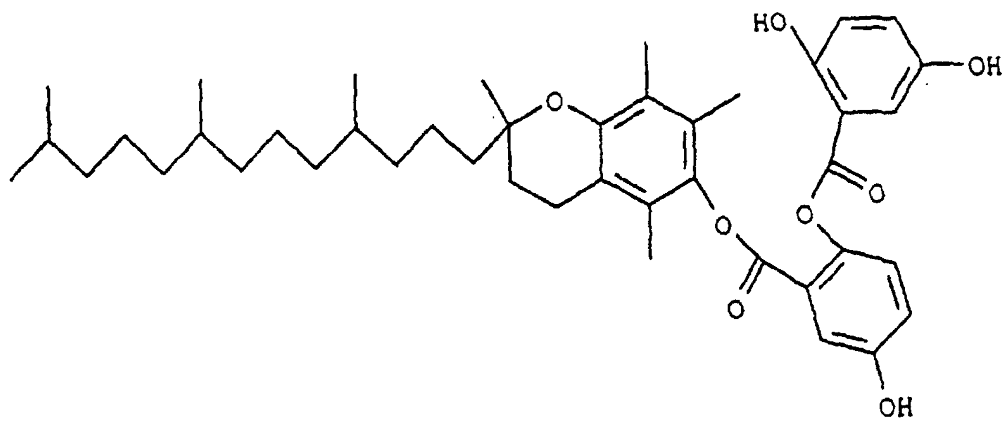

(II)

7. Ester der folgenden Formel (III):

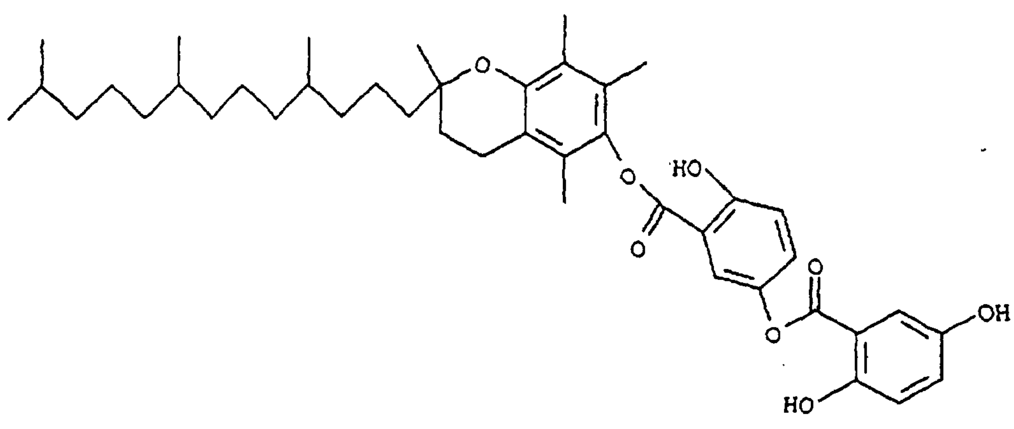

(III)

8. Kosmetische oder pharmazeutische, insbesondere dermatologische Zusammensetzung, **dadurch gekennzeichnet, dass** sie als Wirkbestandteil mindestens einen in einem der vorhergehenden Ansprüche definierten Ester umfasst.

9. Zusammensetzung gemäß Anspruch 8, **dadurch gekennzeichnet, dass** sie als Wirkbestandteil mindestens zwei

gemäß einem der Ansprüche 1 bis 7 definierten Ester in Form einer Mischung umfasst.

**10.** Zusammensetzung gemäß Anspruch 9, **dadurch gekennzeichnet, dass** die Fett-Phase mindestens ein kosmetisch oder pharmazeutisch oder dermatologisch zulässiges Öl umfasst, insbesondere ausgewählt aus der Gruppe aus JojobaÖl, Macadamia-Öl, Öl aus lakrustrischen Kernen, insbesondere aus Limnanthes alba Benth, mineralischen Ölen und aus Triglyceriden, insbesondere aus Triglyceriden auf Basis von Caprin- und/oder Capronsäure, oder aus deren Mischungen.

**11.** Zusammensetzung gemäß einem der Ansprüche 8 bis 10, **dadurch gekennzeichnet, dass** die Konzentration an Ester 0,001 bis 5 und besser noch 0,01 bis 1 Gew.%, bezogen auf das Gesamtgewicht der endgültigen Zusammensetzung, beträgt.

**12.** Zusammensetzung gemäß einem der Ansprüche 8 bis 11, **dadurch gekennzeichnet, dass** sie als Wirkbestandteil mindestens einen α-Tocopherolester der in Anspruch 1 definierten chemischen Formel I umfasst, worin

$R_1$, $R_2$ und $R_3$ jeweils einen Methyl-Rest darstellen,

A die Gruppe:

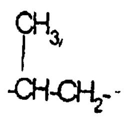

darstellt und

$R_4$ und $R_5$ wie in einem der Ansprüche 1 bis 4 definiert sind.

**13.** Zusammensetzung gemäß einem der Ansprüche 8 bis 12, **dadurch gekennzeichnet, dass** sie mindestens einen weiteren Wirkbestandteil umfasst, insbesondere ausgewählt aus der Gruppe, bestehend aus den Vitaminen A, B1, B6, B12, E, C, PP, Retinsäure, Retinal, Retinol und seinen Estern, Salicylsäure und ihren Derivaten, insbesondere ihren Salzen oder Estern, 2,5-Dihydroxybenzoesäure, Tocopherol und seinen Estern, insbesondere Tocopherolphosphat, Asiatiksäure, Madecassin-Säure und ihren glycosylierten Estern, Asiaticosid, einem Extrakt aus Centella asiatica, einem Extrakt aus Siegesbeckia orientalis, proantocyanidischen Oligomeren und besonders denen aus Traubenkernen, und deren Estern, insbesondere dem Palmitat und Stearat, aus Ascorbinsäure-Derivaten, insbesondere dem Phosphat und ihren Salzen, Erythrobinsäure, aus Oligoelementen und besonders in Form eines Salzes, insbesondere in Form von Aspartat oder Chlorid, wie von Magnesium, Selen, Zink oder Kupfer, aus α-Hydroxysäuren, besonders aus Äpfel-, Milch- und Weinsäure, und deren Estern, insbesondere mit Fettalkoholen, wie mit Stearylalkohol, aus Aminosäuren, insbesondere aus Serin, Threonin, Zitrullin, sowie den Aminosäure-Bestandteilen des NMF, Ceramiden, insbesondere den Ceramiden 2, 3 oder 6, allein oder in Mischung, den Phytoceramiden und insbesondere den aus Weizen extrahierten und aus den Ecdysteroiden, insbesondere Ecdysteron, und deren Estern.

**14.** Zusammensetzung gemäß einem der Ansprüche 8 bis 13, **dadurch gekennzeichnet, dass** der genannte Ester in Kombination mit Vitamin A, vorzugsweise in Form eines Esters wie dem Palmitat, verwendet wird.

**15.** Verwendung mindestens eines in einem der Ansprüche 1 bis 7 definierten Esters als Kosmetikum.

**16.** Verwendung mindestens eines in einem der Ansprüche 1 bis 7 definierten Esters als kosmetisches Mittel zur Vermeidung oder Linderung der schädlichen Wirkungen freier Radikale auf die Haut, zur Vorbeugung oder Behandlung von Rötungen und stechender Gefühle oder von Verbrennungen auf der Haut, zur Begünstigung der Differenzierung der Keratinozyten zur Wiedererstellung einer normalen Hydratation der Epidermis, zur Verbesserung der Feinporigkeit der Haut, zur Verlangsamung oder Behandlung der Alterungeffekte auf die Haut, wie dem Auftreten von Falten oder der Erschlaffung der Haut, und zur Linderung einer Hyperpigmentierung, besonders von Pigment-Flecken wegen alt gewordener Haut.

**17.** Verwendung mindestens eines Esters gemäß einem der Ansprüche 1 bis 7 zur Herstellung einer pharmazeutischen und besonders einer dermatologischen Zusammensetzung mit antiradikalischer, entzündungshemmender Aktivität oder mit anästhetischer Wirkung auf die Nervenendigungen der Haut.

**18.** Verwendung gemäß einem der Ansprüche 15 bis 17, **dadurch gekennzeichnet, dass** der genannte Ester in Form einer Zusammensetzung angewandt wird, umfassend 0,001 bis 5 und noch besser 0,01 bis 8 Gew.% der endgültigen Zusammensetzung.

**19.** Verfahren zur kosmetischen Behandlung, **dadurch gekennzeichnet, dass** man topisch auf die Haut eines Menschen eine kosmetisch wirksame Menge mindestens eines in einem der Ansprüche 1 bis 7 definierten Esters, insbesondere in Form einer Zusammensetzung, enthaltend den vorgenannten Ester in einer Konzentration von 0,001 bis 5 und vorzugsweise von 0,01 bis 1 Gew.% der endgültigen Zusammensetzung, aufträgt, insbesondere zur Vermeidung oder Linderung der schädlichen Effekte freier Radikale auf die Haut, zur Vorbeugung oder Behandlung von Rötungen und stechender Gefühle oder von Verbrennungen auf der Haut, zur Begünstigung der Differenzierung der Keratinozyten, zur Wiedererstellung einer normalen Hydratation der Epidermis, zur Verbesserung der Feinporigkeit der Haut, zur Verlangsamung oder Behandlung der Alterungseffekte auf die Haut, wie dem Auftreten von Falten oder der Hauterschlaffung, und zur Linderung einer Hyperpigmentierung, besonders der Pigment-Flecken wegen alt gewordener Haut.

## Claims

**1.** Esters, **characterized in that** they present the following chemical formula (I):

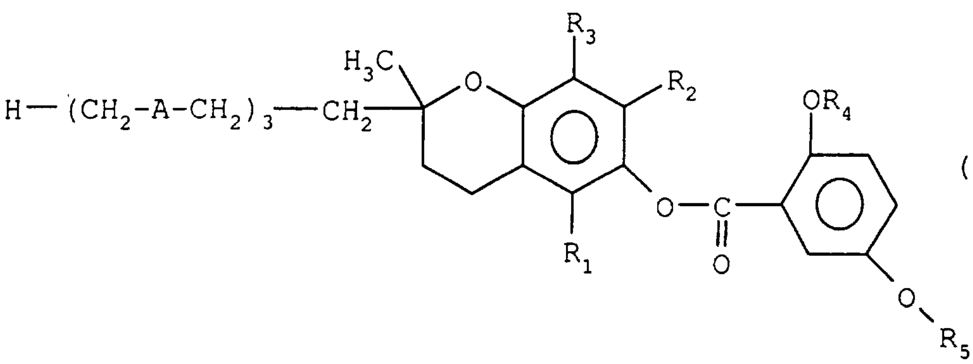

(I)

in which:

$R_1$, $R_2$ and $R_3$ independently represent an atom of hydrogen, a methyl radical,
A represents the following groups:

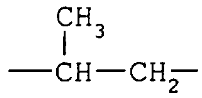, or 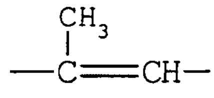,

$R_4$ and $R_5$ are identical or different and each represents a chain of the form:

$$—B_m—C_n—B_p—C_q—H$$

in which:
B is the following group:

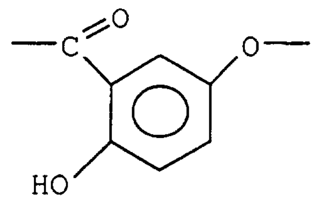

C is the following group:

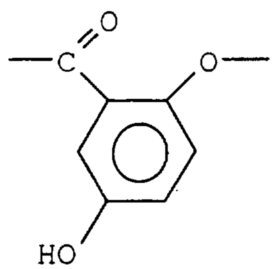

and in which the indices *m*, *n, p*, and *q* are respective integers lying in the range 0 to 4, it being understood that the sum *m* + *n* + *p* + *q* is limited to integers in the range 0 to 4.

2. Esters according to claim 1, **characterized in that** $R_4$ and $R_5$ represent hydrogen.

3. Esters according to claim 1, **characterized in that** at least one of the groups $R_4$ and $R_5$ represents hydrogen.

4. Esters according to claim 3, **characterized in that** one of the groups $R_4$ or $R_5$ represents hydrogen, and the other a 2,5-dihydroxybenzoyl radical.

5. Esters according to any preceding claim, **characterized in that** they are selected from the group consisting in esters of alpha-tocopherol, of beta-tocopherol, of gamma-tocopherol, of zeta 1-tocopherol, of zeta 2-tocopherol, of delta-tocopherol, of eta-tocopherol, of epsilon-tocopherol, and of tocol.

6. An ester satisfying following formula (II):

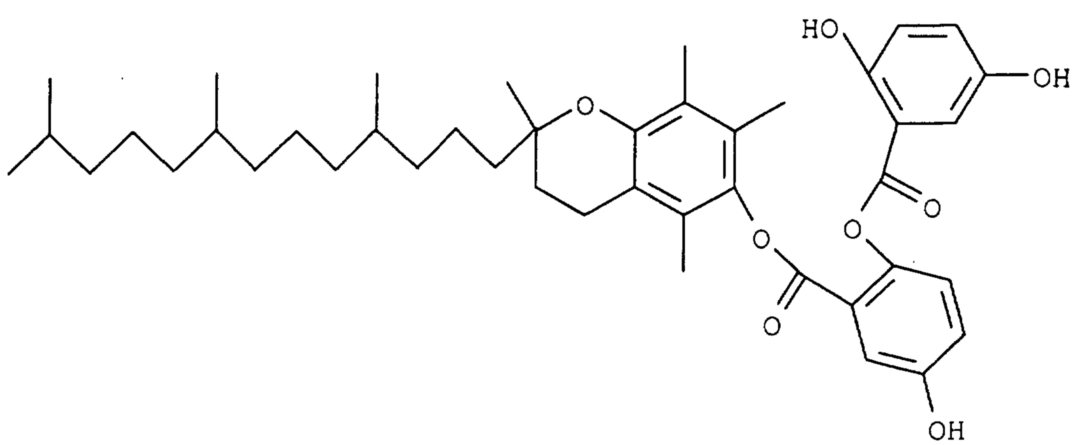

(II)

7. An ester satisfying following formula (III):

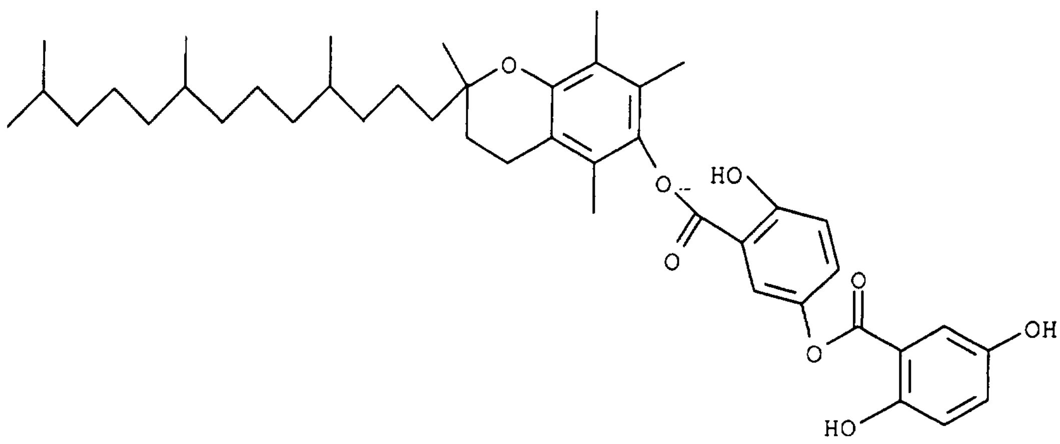

(III)

**8.** A cosmetic or pharmaceutical, in particular dermatological, composition **characterized in that** it comprises as an active ingredient at least one ester as defined in any preceding claim.

**9.** A composition according to claim 8, **characterized in that** it comprises as an active ingredient at least two esters as defined in any one of claims 1 to 7, in the form of a mixture.

**10.** A composition according to claim 9, **characterized in that** the fatty phase comprises at least one cosmetically or pharmaceutically or dermatologically acceptable oil, in particular an oil selected from the group constituted by jojoba oil, macadamia oil, limnanthes seed oil, in particular from *Limnanthes alba Benth,* mineral oils, and triglycerides, in particular triglycerides based on caprylic acid and/or capric acid, or mixtures thereof.

**11.** A composition according to any one of claims 8 to 10, **characterized in that** the ester concentration lies in the range 0.001% to 5%, and better in the range 0.1% to 1% by weight relative to the total weight of the final composition.

**12.** A composition according to any one of claims 8 to 11, **characterized in that** it comprises as an active ingredient at least one alpha-tocopherol ester corresponding to chemical formula I as defined in claim 1, in which each of $R_1$, $R_2$ and $R_3$ represents a methyl radical,

A represents the group:

$$\begin{array}{c} CH_3 \\ | \\ \text{-CH-CH}_2\text{-} \end{array}$$

and $R_4$ and $R_5$ as defined in any one of claims 1 to 4.

**13.** A composition according to any one of claims 8 to 12, **characterized in that** it comprises at least one other active ingredient, in particular selected from the group constituted by vitamins A, B1, B6, B12, E, C, PP, retinoic acid, retinal, retinol and esters thereof, salicylic acid and derivatives thereof, in particular salts or esters thereof, 2,5-dihydroxybenzoic acid, tocopherol and esters thereof, in particular tocopherol phosphate, asiatic acid, madecassic acid and its glycoside-containing esters, asiaticoside, an extract of *Centella asiatica,* an extract of *Siegesbeckia orientalis,* proantocyanide oligomers, in particular those obtained from grape pips, and esters thereof, in particular the palmitic and stearic esters, derivatives of ascorbic acid, in particular its phosphate and its salts, erythorbic acid, oligoelements, in particular in the form of a salt, specifically in the form of an aspartate or a chloride of magnesium, selenium, zinc, or copper, alpha-hydroxylated acids, in particular malic acid, lactic acid, and tartaric acid, and esters thereof, in particular with fatty alcohols, such as stearyl alcohol, amino acids, in particular serine, threonine, citrulline, and amino acids constituting NMF, ceramides, in particular 2, 3 or 6 ceramides used singly or in a mixture, photoceramides, in particular those extracted from wheat, and ecdysteroids, in particular ecdysterone, and esters

thereof.

**14.** A composition according to any one of claims 8 to 13, **characterized in that** said ester is used in combination with vitamin A, preferably in the form of an ester such as palmitate.

**15.** The use of at least one ester as defined in any one of claims 1 to 7 as a cosmetic agent.

**16.** The use of at least one ester as defined in any one of claims 1 to 7 as a cosmetic agent for avoiding or attenuating the harmful effects of free radicals on the skin, for preventing or treating rashes and sensations of skin prickling or stinging, to favor keratinocyte differentiation, to restore normal moisturizing of the epidermis, to improve skin grain fineness, to slow down or treat the effects of aging on the skin, such as the appearance of wrinkles or loose skin, and to attenuate hyperpigmentation, in particular pigmented spots due to skin aging.

**17.** The use of at least one ester according to any one of claims 1 to 7 in the manufacture of a pharmaceutical composition, in particular a dermatological composition, having activity against radicals, against inflammation, or anesthetic action on cutaneous nerve endings.

**18.** The use according to any one of claims 15 to 17, **characterized in that** said ester is used in the form of a composition comprising 0.001% to 5%, or better 0.01% to 1% by weight of the final composition.

**19.** A cosmetic treatment method, **characterized in that** a cosmetically effective quantity of at least one ester as defined in any one of claims 1 to 7 is applied topically to the skin of a human being , in particular in the form of a composition containing said ester at a concentration lying in the range 0.001% to 5%, and preferably in the range 0.01% to 1% by weight of the final composition, in particular, for the purpose of avoiding or attenuating the harmful effects of free radicals on the skin, for preventing or treating rashes and sensations of skin prickling or stinging, to favor keratinocyte differentiation, to restore normal moisturizing of the epidermis, to improve skin grain fineness, to slow down or treat the effects of aging on the skin, such as the appearance of wrinkles or loose skin, and to attenuate hyperpigmentation, in particular pigmented spots due to skin aging.

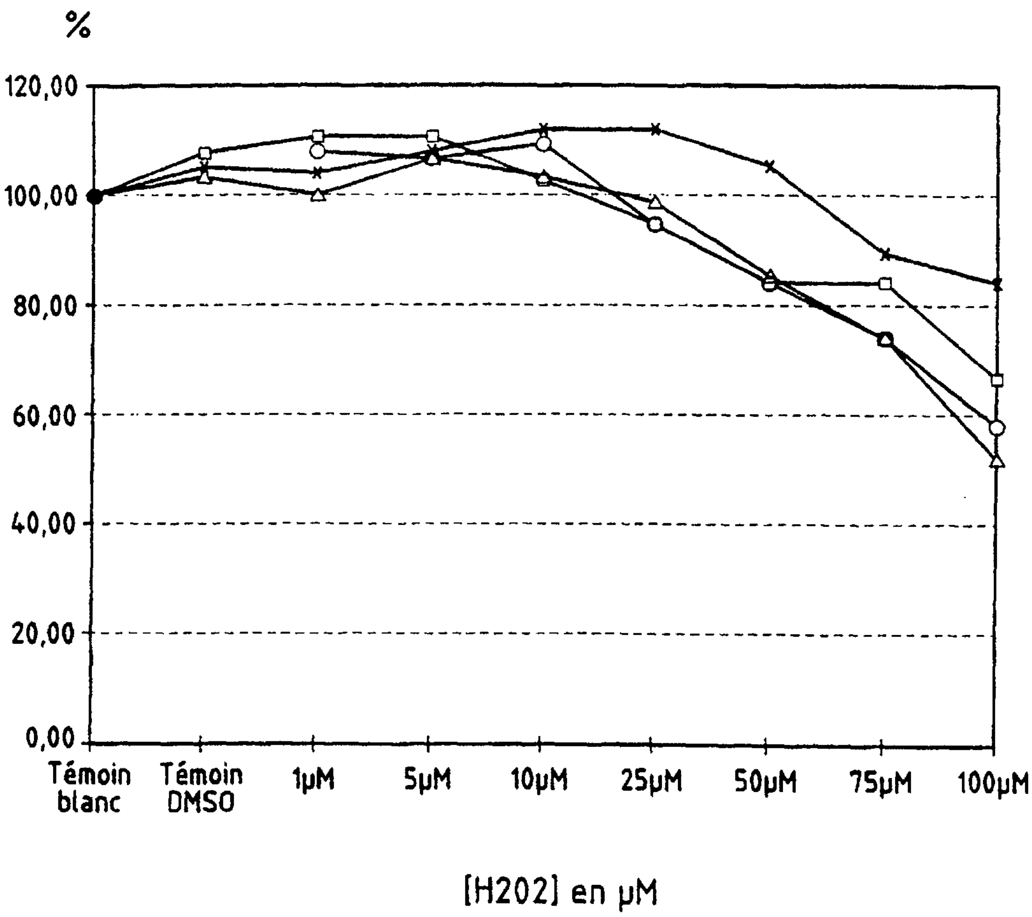
%
120,00
100,00
80,00
60,00
40,00
20,00
0,00
Témoin blanc
Témoin DMSO
1µM
5µM
10µM
25µM
50µM
75µM
100µM
[H2O2] en µM